# EUROPEAN PATENT SPECIFICATION

(11) **EP 3 762 101 B1**
(45) Date of publication and mention of the grant of the patent: **06.10.2021**
(21) Application number: 19708548.3
(22) Date of filing: 07.03.2019
(51) Int. Cl.: A61N 5/10, A61B 6/00, G01B 11/00, G01B 11/27, A61B 6/08, A61B 6/04

(54) **A DEVICE CONFIGURED TO BE ALIGNED WITH RESPECT TO A MEDICAL SYSTEM**
VORRICHTUNG ZUR AUSRICHTUNG IM HINBLICK AUF EIN MEDIZINISCHES SYSTEM
DISPOSITIF CONÇU POUR ÊTRE ALIGNÉ PAR RAPPORT À UN SYSTÈME MÉDICAL

(30) Priority: 08.03.2018 EP 18160730
(43) Date of publication of application: 13.01.2021
(73) Proprietor: Koninklijke Philips N.V., 5656 AE Eindhoven (NL)
(72) Inventor: NOUSIAINEN, Jere, Matti, 5656 AE Eindhoven (NL); SOININEN, Mikko, Aleksi, 5656 AE Eindhoven (NL)
(74) Representative: Philips Intellectual Property & Standards
(86) International application number: PCT/EP2019/055627
(87) International publication number: WO 2019/170786

(56) References cited:
- WO-A1-2016/058813
- WO-A2-00/65396
- JP-A- 2008 113 803

## Description

### FIELD OF THE INVENTION

This invention relates to the field of medical systems and more specifically to medical imaging and / or medical treatment system. Even more specifically, this invention relates to medical imaging and / or medical treatment systems in the field of radiotherapy.

### BACKGROUND OF THE INVENTION

Alignment of patients or other objects by means of a light source, preferably emitting high intensity light, like e.g. a laser system is commonly used in medical systems, like e.g. treatment and imaging systems. Especially, in the field of treatment accurate alignment of patients or objects is of high importance. For quality assurance of medical systems, often phantoms are used.

In standard clinical practice, an object is aligned by means of a cross laser beam, wherein milled or painted lines on the object are aligned with the laser light.

JP2008113803 describes a method and device for aligning phantoms, wherein light is guided through a phantom.

### SUMMARY OF THE INVENTION

It is an object of the invention to provide for an improved device that is configured to be aligned with a medical system. This object is achieved by a device according to claim 1.

The device as proposed in claim 1 comprises at least one optical fiber. The at least one optical fiber extends from an alignment position to an indicator position. The at least one optical fiber is configured to guide light provided by the light source from the alignment position to the indicator position in case the device is correctly aligned with respect to the medical system and light source. Only in case of correct alignment, the light will enter the optical fiber at the alignment position and as a result only in these situations the light will be transmitted to the indicator position of the optical fiber. In this way by looking at the indicator end of the optical fiber, it can more easily be seen by the user that the alignment of the device with respect to the medical system is correct.

Compared to JP2008113803, the invention provides a lot of freedom in device (e.g. phantom) design, because the device does not need to be transparent nor does it need to comprise a hole. Detection of correct aligment does not need to take place near the part of the device that has to be aligned. The optical fibers could be guided to location more remote from the part of the device that has to be aligned, e.g. to an entrance of the medical system or even to another room. This may result in workflow benefits. Another advantage of the current proposal is that no additional sensors are needed.

Preferably, the medical system is a medical imaging system and / or a treatment system. Examples of such systems could be radiotherapy systems, MRI systems, (cone-beam) CT systems, PET systems.

Preferably, the light source is a laser system, as these systems are mostly used in the present clinical practise to align objects and patients with medical systems.

The device could for example be a phantom, a holder, wherein a phantom can be positioned, a patient alignment tool, a patient support, or a servicing tool configured for use in servicing of the medical system, e.g. to align different parts of the medical system with respect to each other.

According to embodiments of the invention, a fiber end at the alignment position is substantially perpendicular to an expected direction of light provided by the light source, when the device is correctly aligned with the medical system and light source.

According to further embodiments of the invention, the device comprises a light entrance area from which light provided by the light source can enter the optical fiber on the end of the alignment position. The light entrance area has a width of equal to or less than a desired positioning precision. This can for example be achieved by using an optical fiber with a thickness equal to or less than the desired positioning precision. Also, arrangements may be provided at the alignment position end of the optical fiber, that partly block the light from the light source, such that the light can only enter the optical fiber in a light entrance area having a width of equal to or less than the desired positioning precision. This embodiment is advantageous because it allows to adapt the accuracy of the alignment to the requirements of a specific situation. The smaller the light entrance area, the more accurate the alignment result. However, a disadvantage is that a smaller light entrance area provides a less bright light signal at the indicator position. Therefore, according to embodiments of the invention for small light entrance areas (e.g. substantially smaller than 1 mm width) the device further comprises a lens, wherein the lens is configured to increase an intensity of the light traveled through the optical fiber at the indicator position.

Additionally or alternatively, a lens may be used to focus the light on the side of the alignment position. This is especially advantageous when (laser) light has to travel a substantial distance before reaching the alignment position of the optical fiber.

When the device comprises a single optical fiber, it can be used to align the device in a single direction, e.g. the z-direction. This could for example be useful when two objects have to aligned on the same height. However, commonly in clinical practise a device has to be aligned in multiple directions. Therefore, according to embodiments of the invention, the device comprises multiple optical fibers.

The use of multiple optical fibers could also help to improve the alignment workflow. According to embodiments of the invention, the device comprises a target optical fiber configured to indicate correct alignment. In addition to that the device further comprises at least one guiding optical fiber configured to indicate that the device is slightly misaligned, wherein the alignment position of the guiding optical fiber is positioned substantially near the alignment position of one of the target optical fiber. This embodiment is advantageous, because it can help the user in determining how he should change the position of the device in order to obtain correct alignment.

These and other aspects of the invention will be apparent from and elucidated with reference to the embodiments described hereinafter.

### BRIEF DESCRIPTION OF THE DRAWINGS

Fig. 1 diagrammatically shows a device according to the prior art and
Fig. 2 diagrammatically shows a device according to embodiments of the invention and
Fig. 3 diagrammatically shows a device according to another embodiment of the invention
Fig. 4 diagrammatically shows a device comprising a single optical fiber.

### DETAILED DESCRIPTION OF E

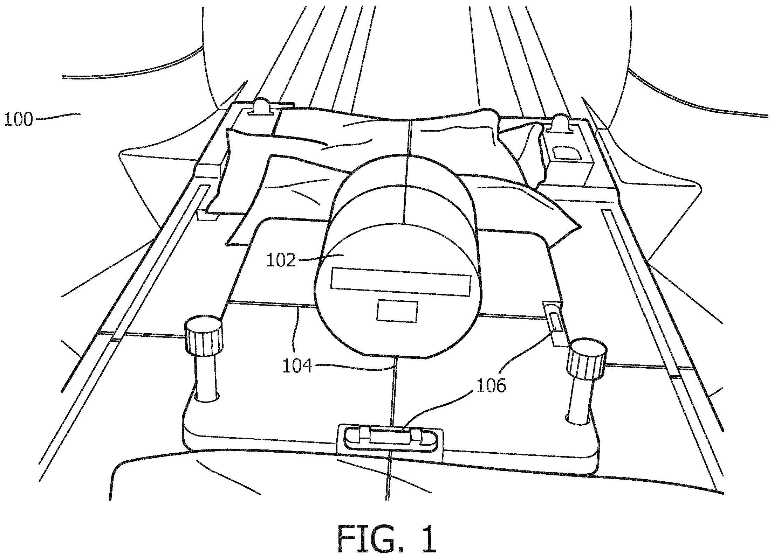

Fig. 1 diagrammatically shows a device according to the prior art. The device 102 has to be aligned with medical system 100, which in this figure is an MRI system. For the alignment laser light 104 provided by a laser system is used. The device is aligned correctly with respect to the imaging system when the laser light goes through grooves 106. Although the device is aligned at the entrance of the medical system, this is not the location where the accuracy of the positioning needs to be known. It is assumed that when alignment of the device with respect to the medical system is correct at the entrance of the medical system, this alignment remains correct when the device is deliberately moved to the location where it will be imaged or treated. 'Treated' in this respect shall be interpreted as being exposed to the output of a treatment system (e.g. radiation in case of a radiotherapy system)

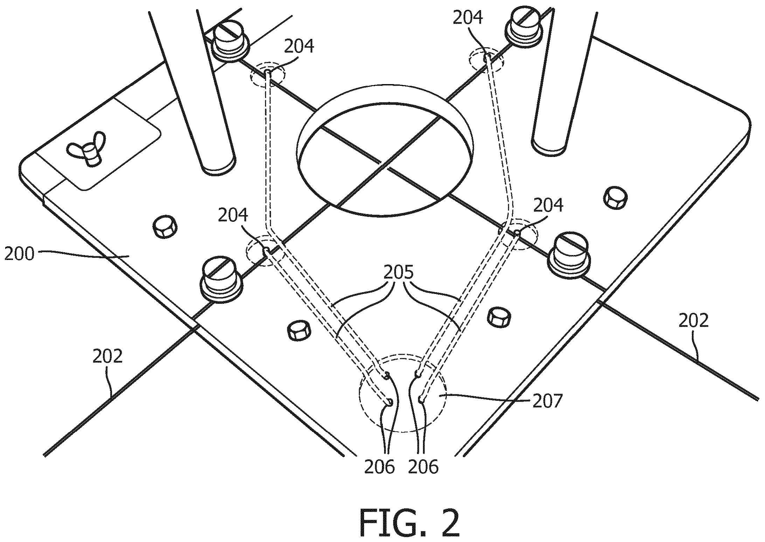

Fig. 2 shows a device 200 according to embodiments of the invention. The device is configured to be aligned by means of two laser beams 202. When the device is positioned correctly with respect to the laser system and the medical system, the laser light will hit the alignment positions 204 of optical fibers 205 and enter the optical fiber. As can be seen, when the device is positioned correctly, the alignment positions 204 of the optical fibers 205 are substantially perpendicular to the direction of the laser beam. Only in case the laser light hits / enters at the alignment positions 204, the optical fibers guide the laser light to the respective indicator positions 206. When laser light appears at the indator positions, the user of the device knows that the device has been positioned correctly. Fig. 2, illustrates an advantage of the present invention. Using optical fibers is advantageous, because in this way it is possible to avoid the need of a user to go around the device to check if it is aligned correctly, e.g. in fig. 1 and the state of the art the grooves are distributed around the device, whereas the indicator positions when using the invention can be placed on a small area, e.g. fig. 2, 207. This provides workflow advantages. Also optical fibers make it more easy to perfrom a position alignment at the location of imaging or treatment. As can be seen in fig. 1, it will be difficult to align the state of the art device within the medical system 100 itself. In contrast a device according to embodiments of the invention could be aligned at the position of treatment or imaging (e.g. within the medical system itself), because embodiments of the invention provide for the flexibility to guide the optical fibers to a location remote from the part of the device that has to be imaged or treated. Depending on the desired alignment position, the thickness of the one or more optical fibers can be varied. Thinner optical fibers can be used when a higher precision is needed. As an alternative also means can be used to partially block the light that can enter the optical fiber. For example (small) plates or blocks could be used for this. Also, the alignment position of the optical fiber could be surrounded by non-transparent material having a (small) hole through which light may enter. Many alternatives are possible and known to the skilled person. For small light entrance areas (e.g. having a width well below 1 mm), it may be preferable to use a lens 210 to intensify the light spot at the indicator position.

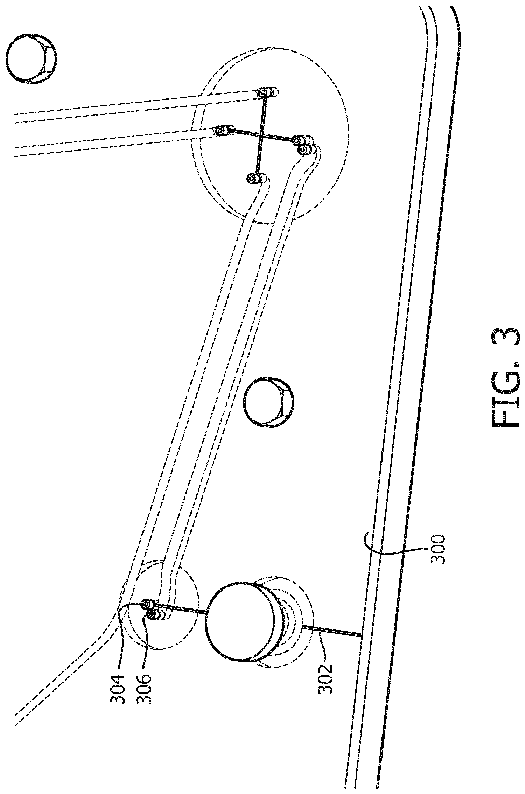

Fig. 3 diagrammatically shows a device 300 according to another embodiment of the invention. The device comprises a target optical fiber 304 configured to indicate correct alignment. The target optical fiber has a similar function as the optical fibers described with respect to fig. 2. The device further comprises at least one guiding optical fiber 306 configured to indicate that the device is slightly misaligned. As can be seen the alignment position of the guiding optical fiber is positioned substantially near the alignment position of one of the target optical fiber. When the light from the light source hits the alignment position of the guiding optical fiber, this will be indicated at the indicator position of the guiding optical fiber. Hereby, the user will know that the alignment is close to being correct, but is not correct yet. Line 302 shows how the light will hit the device when the device is positioned correctly.

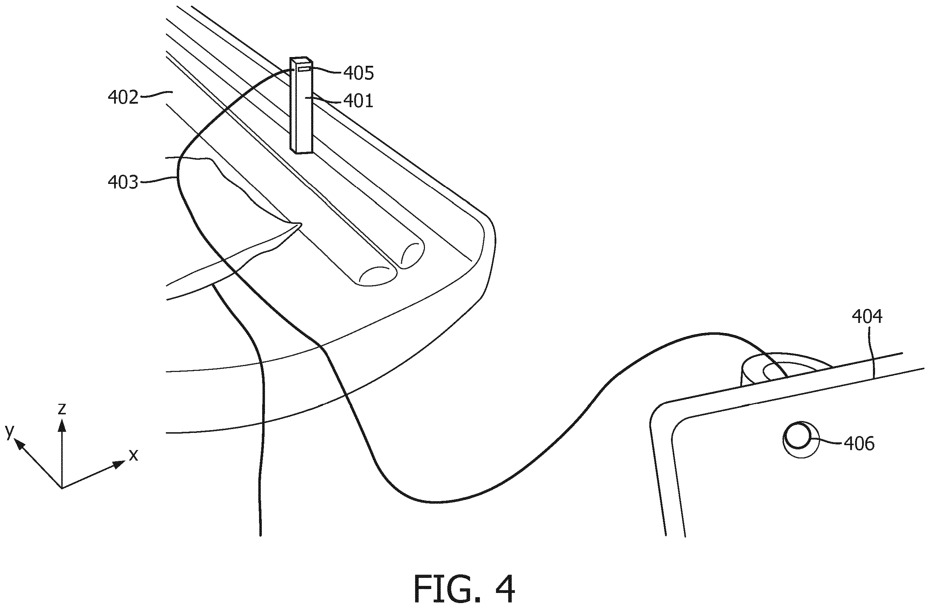

Fig. 4 diagrammatically shows a device comprising a single optical fiber. This device can be used in order to align the device or the object it is attached to in a single direction, in this case the z-direction (height). In the embodiment depicted in fig.4, a first holder 401 is attached to a patient support 402. The device intended to be used to position the patient table on the correct height. The optical fiber extends from the alignment position 405 on the first holder 401 to an indicator position 406. The indicator position is on the end of the optical fiber. The indicator is preferably attached to a second holder 404. The optical fiber or the second holder could be held by a user. In this way the user could easily see if the device and the object it is attached to are positioned on the intended height.

In addition, the device can be used to take care that the object it is attached to (e.g. the patient support 402) is positioned at the same height over its entire length, i.e. to take care that it is not tilted. This can be done by repositioning the first holder at a different location along the y-axis, which is parallel to the laser beam and repeating the procedure.

Whilst the invention has been illustrated and described in detail in the drawings and foregoing description, such illustrations and description are to be considered illustrative or exemplary and not restrictive; the invention is not limited to the disclosed embodiments.

## Claims

1. A device configured for being aligned with respect to a medical system by means of a light source, **characterized in that** the device comprises at least one optical fiber, wherein the at least one optical fiber extends from an alignment position to an indicator position and wherein the at least one optical fiber is configured to guide light provided by the light source from the alignment position to the indicator position in case the device is correctly aligned with respect to the medical system and light source.

2. A device as claimed in claim 1, wherein a fiber end at the alignment position is substantially perpendicular to an expected direction of light provided by the light source, when the device is correctly aligned with the medical system and light source.

3. A device as claimed in any of the preceding claims, comprising a light entrance area from which light provided by the light source can enter the optical fiber on the end of the alignment position, wherein the light entrance area has a width of equal to or less than a desired positioning precision.

4. A device as claimed in any of the preceding claims comprising multiple optical fibers.

5. A device as claimed in claim 4, wherein the device comprises a target optical fiber configured to indicate correct alignment and wherein the device further comprises at least one guiding optical fiber configured to indicate that the device is slightly misaligned, wherein the alignment position of the guiding optical fiber is positioned substantially near the alignment position of one of the target optical fiber.

6. A device as claimed in any of the preceding claims, wherein the device is one out of a phantom, a holder, wherein a phantom can be positioned, a patient alignment tool, a patient support, or a servicing tool configured for use in servicing of the medical system.

7. A device as claimed in any of the preceding claims, wherein the light source is a laser system.

8. A device as claimed in any of the preceding claims further comprising a lens, wherein the lens is configured to increase an intensity of the light traveled through the optical fiber at the indicator position.

## Patentansprüche

1. Ein Gerät, das mithilfe einer Lichtquelle in Bezug auf ein medizinisches System ausgerichtet werden kann, **dadurch gekennzeichnet, dass**
das Gerät mindestens einen Lichtwellenleiter umfasst, wobei sich der mindestens eine Lichtwellenleiter von einer Ausrichtungsposition zu einer Anzeigeposition erstreckt, und wobei der mindestens eine Lichtwellenleiter das von der Lichtquelle bereitgestellte Licht von der Ausrichtungsposition zur Anzeigeposition leitet, wenn das Gerät ordnungsgemäß auf das medizinische System und die Lichtquelle ausgerichtet ist.

2. Ein Gerät gemäß Anspruch 1, wobei ein Lichtwellenleiterende in der Ausrichtungsposition im Wesentlichen senkrecht zur erwarteten Richtung des von der Lichtquelle bereitgestellten Lichts ist, wenn das Gerät ordnungsgemäß am medizinischen System und der Lichtquelle ausgerichtet ist.

3. Ein Gerät gemäß einem der vorherigen Ansprüche, das einen Lichteintrittsbereich umfasst, aus dem das von der Lichtquelle bereitgestellte Licht am Ende der Ausrichtungsposition in den Lichtwellenleiter eintreten kann,
wobei der Lichteintrittsbereich eine Breite aufweist, die höchstens der gewünschten Positioniergenauigkeit entspricht.

4. Ein Gerät gemäß einem der vorherigen Ansprüche, das mehrere Lichtwellenleiter umfasst.

5. Ein Gerät gemäß Anspruch 4, wobei das Gerät einen Ziellichtwellenleiter umfasst, der die ordnungsgemäße Ausrichtung angibt, und wobei das Gerät zudem mindestens einen Führungslichtwellenleiter umfasst, der angibt, dass das Gerät geringfügig falsch ausgerichtet ist, wobei sich die Ausrichtungsposition des Führungslichtwellenleiters im Wesentlichen in der Nähe der Ausrichtungsposition eines der optischen Ziellichtwellenleiter befindet.

6. Ein Gerät gemäß einem der vorherigen Ansprüche, wobei es sich beim Gerät um ein Phantom, eine Halterung, in der ein Phantom positioniert werden kann, eine Patientenausrichtungsvorrichtung, eine Patientenauflage oder ein Wartungswerkzeug zum Warten des medizinischen Systems handelt.

7. Ein Gerät gemäß einem der vorherigen Ansprüche, wobei es sich bei der Lichtquelle um ein Lasersystem handelt.

8. Ein Gerät gemäß einem der vorherigen Ansprüche, das zudem eine Linse umfasst, wobei die Linse die Intensität des durch den Lichtwellenleiter zur Anzeigeposition geleiteten Lichts erhöht.

## Revendications

1. Dispositif conçu pour être aligné par rapport à un système médical au moyen d'une source lumineuse, **caractérisé en ce que**
le dispositif comprend au moins une fibre optique, dans lequel l'au moins une fibre optique s'étend d'une position d'alignement à une position d'indicateur et dans lequel l'au moins une fibre optique est conçue pour guider la lumière fournie par la source lumineuse de la position d'alignement à la position d'indicateur lorsque le dispositif est correctement aligné par rapport au système médical et à la source lumineuse.

2. Dispositif selon la revendication 1, dans lequel une extrémité de fibre au niveau de la position d'alignement est sensiblement perpendiculaire à une direction de lumière attendue fournie par la source lumineuse, lorsque le dispositif est correctement aligné par rapport au système médical et à la source lumineuse.

3. Dispositif selon l'une quelconque des revendications précédentes, comprenant une zone d'entrée de lumière à partir de laquelle la lumière fournie par la source lumineuse peut entrer dans la fibre optique à l'extrémité de la position d'alignement, dans lequel la zone d'entrée de lumière présente une largeur égale ou inférieure à une précision de positionnement souhaitée.

4. Dispositif selon l'une quelconque des revendications précédentes, comprenant de multiples fibres optiques.

5. Dispositif selon la revendication 4, dans lequel le dispositif comprend une fibre optique cible conçue pour indiquer un alignement correct, et dans lequel le dispositif comprend en outre au moins une fibre optique de guidage conçue pour indiquer que le dispositif est légèrement désaligné, dans lequel la position d'alignement de la fibre optique de guidage est positionnée sensiblement près de la position d'alignement de l'une des fibres optiques cibles.

6. Dispositif selon l'une quelconque des revendications précédentes, dans lequel le dispositif fait partie d'un fantôme, d'un support, dans lequel un fantôme peut être positionné, d'un outil d'alignement de patient, d'un support de patient ou d'un outil d'entretien conçu pour être utilisé dans l'entretien du système médical.

7. Dispositif selon l'une quelconque des revendications précédentes, dans lequel la source lumineuse est un système laser.

8. Dispositif selon l'une quelconque des revendications précédentes comprenant en outre une lentille, dans lequel la lentille est conçue pour augmenter une intensité de la lumière parcourue à travers la fibre optique au niveau de la position d'indicateur.
